Europäisches Patentamt

European Patent Office

Office européen des brevets

(11) Publication number: **0 314 657**
**A1**

## EUROPEAN PATENT APPLICATION

(21) Application number: **88850362.0**

(22) Date of filing: **25.10.88**

(51) Int. Cl.⁴: **A 61 B 1/24**
**A 61 C 17/04**

(30) Priority: **28.10.87 SE 8704186**

(43) Date of publication of application:
**03.05.89 Bulletin 89/18**

(84) Designated Contracting States:
**AT BE CH DE ES FR GB GR IT LI LU NL SE**

(71) Applicant: **INVO AB**
**Eliselundsvägen 18**
**S-270 57 Kivik (SE)**

(72) Inventor: **Gullstrand, Lars**
**Eliselundsvägen 18**
**S-270 57 Kivik (SE)**

(74) Representative: **Asketorp, Göran P.**
**Asketorp Patent & Juridik AB P.O. Box 1**
**S-230 10 Skanör (SE)**

(54) **A dental mirror.**

(57) A dental mirror comprising a shaft (2) and a head (3) having a mirror (4) arranged in a brace (6). The shaft has a boring (26), connected to a source of underpressure. The boring opens centrally below the mirror under a hole (13) in the brace. The brace is provided with shoulders (8) for holding the mirror and forming slits around the periphery of the mirror for connecting the boring with the space outside the mirror for suction of air, liquid and smaller particles through said slits to the boring and further to the source of underpressure. Said flow of air/liquid prevents formation of moisture and removes air/liquid-bubbles on the mirror.

Fig. 3

EP 0 314 657 A1

Bundesdruckerei Berlin

**Description**

## A DENTAL MIRROR

### FIELD OF INVENTION

The present invention relates to a dental mirror having an integrated evacuation suction tube.

### PRIOR ART

Dental mirrors are well-known and the dental mirror mostly used comprises a small circular mirror attached to a shaft. The mirror is attached at an angle in order to give the best observation position. If the mirror is too large, it is unconvenient for the patient and cumbersome for the dentist.

Most dentists of today use a saliva ejector placed at the bottom of the mouth and a straight or angled evacuation tube for suction adjacent the tooth being treated with a dentist's drill and sprayed with water.

A drawback of ordinary dental mirrors is that they will be covered with moisture and liquid/air-bubbles and small particles from removed material, which prevents the dentist in his work and the mirror must be mechanically wiped off rather often.

In the art it has been suggested to combine the dental mirror and the suction tube into a single instrument.

In Swedish Patent No. 8501123-7 there is disclosed such an instrument having a shaft and a mirror covering a cupshaped head attached to the shaft. The edge of the head is provided with several holes for suction of air/liquid and small particles. A separate air jet is directed towards the upper surface of the mirror in order to keep it free from moisture.

In German Offenlegungsschrift No. 28 44 774, there is disclosed a similar dental instrument having a shaft and a head covered with a mirror. The mirror is centrally attached to the head at a small distance thereabove, so that a narrow slit is formed between the edge of the head and the bottom edge of the mirror for suction of air/liquid and small particles.

US Patent No. 2,861,342 also discloses a dental mirror having a slit at the side of a head attached to a shaft.

US Patent No. 3,928,916 discloses a dental mirror having holes in the bottom of a cupshaped head covered by the mirror. Moreover, there is provided a slit between the edge of the mirror and the edge of the head for suction of particles.

### SUMMARY OF INVENTION

The object of the present invention is to suggest a combination or integration of a dental mirror and a suction tube as shown in the patents mentioned above. Then, the advantage is obtained that the ejection of air/liquid and small particles can take place at a desired position by simple moving the mirror to that place, i.e. the mirror and the suction tube can be handled at the same time.

More specifically, the object of the present invention is to provide a dental mirror of the above-mentioned type in which the formation of moisture on the surface of the mirror is prevented.

It is observed that the formation of moisture on the mirror depends on the fact that the mirror surface is exposed to a humide air-flow and the mirror is often colder than the air temperature and form a cold surface where the humidity will condense.

According to the present invention, the flow of air/liquid through the head of the dental mirror is used for heating the mirror. Moreover, the flow of air/liquid is directed close to the upper surface of the mirror to carry away moisture that could otherwise condense on the surface of the mirror. By a combination of these two messures, a dental mirror is provided that is self-cleaning as to moisture and particles on the surface of the mirror. Moreover, liquid bubbles are often gathered on the surface of the mirror. By direction of the air-flow close and parallel to the upper surface of the mirror, such bubbles are also removed.

In order to achieve the above-mentioned objects, the dental mirror according to the present invention is provided with several slits around the edge of the mirror. A first slit directs the flow of air/liquid essentially parallel with the surface of the mirror, a second slit directs the flow around the edge of the mirror to the back-side of the mirror and a third slit directs the flow parallel with and close to the back-side of the mirror to a central hole below the mirror and further to the shaft of the dental mirror. A conventional suction tube is connected to the shaft and is removable from the dental mirror for separate use. The saliva ejector at the bottom of the mouth should be used as previously.

Thus, there is provided a dental mirror comprising a shaft and a head covered by a mirror. The shaft is provided with a boring, which is connected to a suction tube. The boring opens below the mirror at a central hole. Slits are arranged below the mirror and around the periphery thereof in order to connect the boring with the space outside the mirror for suction of air/liquid and smaller particles through said slits and to the boring and further to the suction tube.

Preferably, the mirror is positioned in a brace having shoulders forming spacing means for forming said slits. The brace is positioned in the head of the dental mirror. The shoulders form a first slit between the upper surface of the mirror and a separate ring retaining the mirror, a second slit between a wall of the head and the periphery of the mirror and a third slit between the underside of the mirror and an upper surface of the brace. The brace is provided with a central hole in the bottom surface thereof opening to a channel leading to the boring.

According to a preferred embodiment of the invention, the boring is divided in two portions, an upper somewhat conical portion having a large diameter and a lower portion having a small diameter. The suction tube is connected to said upper boring portion with a frictional fit. The transition between the two boring portions forms a shoulder for stopping the insertion of the suction tube in the boring. Preferably, the shaft has an outer surface in the shape of a polygon, preferably an octagon.

## SHORT DESCRIPTION OF THE DRAWINGS

The invention is described below in more details with reference to the appended drawings.

Fig. 1 is a perspective view of the dental mirror according to the invention.

Fig. 2 is an exploded view in perspective of the dental mirror according to Fig. 1.

Fig. 3 is a longitudinal section in an enlarged scale through the dental mirror according to Fig. 1.

Fig. 4 is an exploded view in cross-section of the dental mirror according to Fig. 2.

Fig. 5 is a longitudinal section in a further enlarged scale of the area encircled by line V in Fig 3.

## DETAILED DESCRIPTION OF A PREFERRED EMBODIMENT

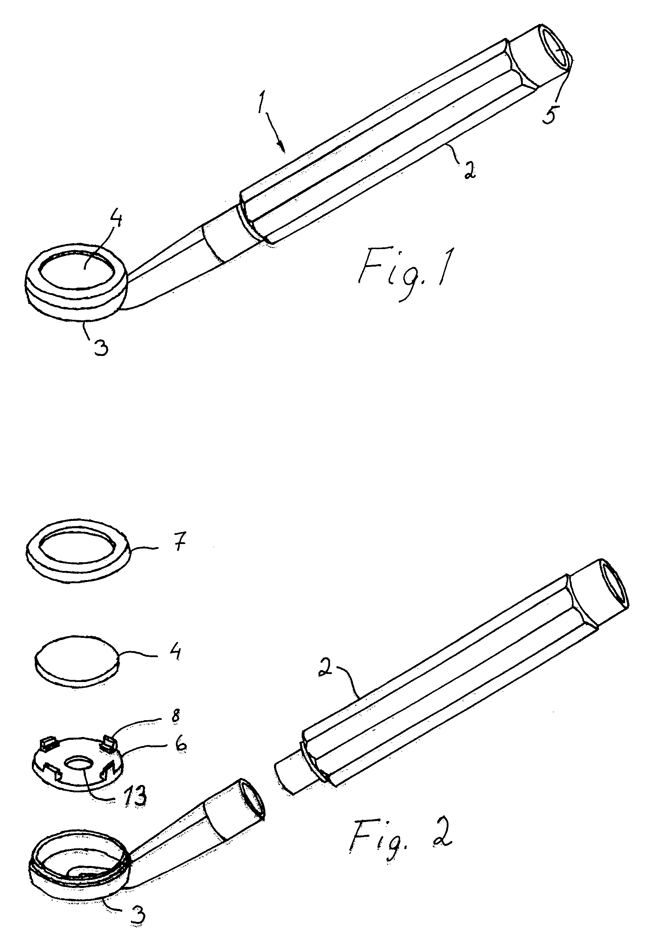

A dental mirror according to the invention is shown in perspective in Fig. 1. The dental mirror 1 comprises a shaft 2 and a head 3, in which a mirror 4 is attached. The shaft 2 is relatively thick and is hollow. The outer end of the shaft is provided with an opening 5.

The shaft is octagonal, but any polygon can be used. The shaft can also be cylindrical. However, by the polygonal shape, the advantage is obtained that the grip will be firmer. Moreover, the thick shaft fits easy in the hand and gives an ergonomically correct handling of the instrument.

With reference to Fig. 2, the dental mirror 1 comprises five separate parts, the shaft 2, the head 3, the mirror 4 and a holder for the mirror in two parts, a brace 6 and a retaining ring 7. The shaft 2 and the head 3 are interconnected by a sleeve fitting, either with a friction fit, by a suitable conicity or by gluing or similar.

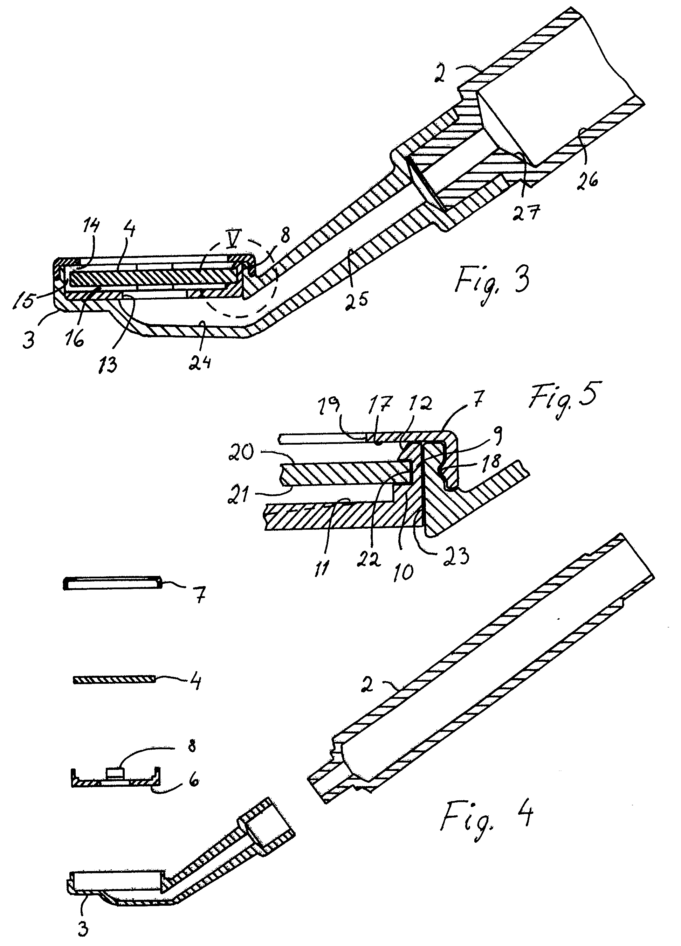

The brace 5 is provided with four shoulders 8 with the cross-section shown in details in Fig. 5. Each shoulder 8 has a vertical portion 9 and a horizontal portion 10. The horizontal portion 10 spaces the mirror 4, when inserted in the brace 6, above the upper surface 11 of the brace between the shoulders 8. The vertical portion 9 has a hook 12 that retains the mirror when inserted in the brace.

The brace with the mirror is inserted in the head 3 of the dental mirror in a recess. The retaining ring 7 is fitted over the head and is hold in place by a snap connection 18 shown in Fig. 5.

A first slit 14 parallel with the upper surface of the mirror is formed between the lower surface 17 of the ring 7 and the upper surface 20 of the mirror 4. The length of the first slit is determined by the open area 19 of the ring 7. Preferably, the area of the opening 19 is from 40 to 90% of the area of the mirror, preferably around 65%, which gives a sufficient length of the first slit. If the mirror has a diameter of 20 mm, then the length of the first slit is from 1 to 3 mm and perferably 2 mm. The height of the slit is around 1 mm.

A second slit 15 is provided between the periphery 22 of the mirror and the inner wall 23 of the head 3 and perpendicular to the first slit. The dimension of this slit is not essential as soon as it is not narrower than the first slit. Preferably, it has the same height as the first slit.

A third slit is formed between the lower surface 21 of the mirror and the upper surface 11 of the brace 6 and parallel with the fist slit. The height of the third slit is essentially the same as the first and second slits but it might be enlarged as shown by the dotted line in Fig. 5. The third slit extends to the central hole 13 of the brace 6.

Thus, there is provided a narrow flow path around the periphery of the mirror and further below the lower side of the mirror to the central hole 13. The hole 13 opens to a channel 24 in the bottom of the head leading to a boring 25 in the head portion and to a boring 26 in the shaft 2.

A conventional evacuation suction tube (not shown) is inserted in the boring 26 through the opening 5 and fastened by friction due to a small conicity of the boring 26. The suction tube also fastens due to the underpressure prevailing in the suction tube.

A shoulder 27 formed between the upper portion and the lower portion of the boring 26 stops further insertion of the suction tube.

From the above description it is evident that a flow of air/liquid and small particles takes place from the area outside the dental mirror to the suction tube when connected to a source of underpressure. The flow through the first slit is essentially parallel with the surface 20 of the mirror and will remove bubbles, particles and moisture from the mirror surface in the exposed central area 19. Thence, the flow is deflected 180° via the second slit and passes below the mirror in the third slit. In said third slit the flow heats the mirror to a temperature close to the surrounding atmosphere, which further prevents the formation of moisture on the upper surface of the mirror. It is believed that it is the combination of heating the mirror essentially in the third slit and the physical removing of the moisture before the first slit which makes it possible to achieve a dental mirror that is free from moisture at the surface of the mirror.

It is of great importance that the flow is essentially equally distributed over the entire periphery of the mirror. Thus, it is important that the slits are distributed over the entire periphery of the mirror (except the shoulders) and are as uniform as possible. Moreover, it is of importance that the hole 13 is positioned at the centre of the brace so that the suction pressure is distributed uniformly over the periphery of the mirror. Thus, the shape and dimensions of the brace 6 are of great importance for the satisfactory operation of the invention.

In Fig. 2 the brace is shown rotated 45° around its own axle in relation to the shaft while in Figs. 3 and 4 the same angle is 0°. Said rotation of the brace is unimportant since the suction still takes place centrally via the hole 13.

The suction tube is retained merely by friction and is releasable. If larger particles which cannot pass through the slit should be removed, the suction tube is released and is used in a conventional manner.

Since liquid and small particles flow below the reflecting surface of the mirror, it is possible that the mirror layer will erode and it might be necessary to protect it by a layer of plastics or varnish or another hydrofobic material. Thus, the surface over which the liquid flows will also be smoother and a stronger flow may be obtained. Alternatively, a double mirror may be used in which the mirror layer is protected by the glass surface facing downward.

The invention has been described above with reference to an embodiment shown on the drawings. However, said embodiment can be modified in many respects within the scope of the invention. Such modifications obvious to a skilled person are intended to be within the scope of the invention. The invention is only limited by the patent claims below.

somewhat conical portion (26) having a large diameter, in which portion a suction tube fits with friction fitting, a lower portion having smaller diameter, the transition between these two portions forming a shoulder (27) for preventing further insertion of the suction tube in the boring (26), and a channel (24) leading to said hole (13).

**Claims**

1. A dental mirror comprising a shaft (2) and a head (3) having a mirror (4), the shaft being provided with a boring (26) connected to a source of underpressure and the boring (26) opening to a cupshaped recess below the mirror (4) centrally under the mirror (4), said recess being provided with slits for suction of a flow of air, liquid and small particles through said slits to the boring and further to the source of underpressure, **characterized** in that the slits are positioned essentially along the entire periphery of the mirror and comprises

a first slit (14) between the upper surface of the mirror and a ring (7) extending from the periphery of the mirror and towards the central portion thereof parallel to the surface of the mirror,

a second slit (15) essentially perpendicular to the first slit and positioned between the periphery of the mirror and the edge of the head, and

a third slit (16) between the underside of the mirror and a surface (11) of the head, and

a centrally positioned hole (13),

whereby said flow passes essentially uniform over the outer surface of the mirror, around the periphery thereof through the second slit, and below the mirror towards the middle thereof in the third slit and further through said hole to said boring.

2. A dental mirror according to claim 1, **characterized** in that the mirror (4) is positioned in a brace (6) having shoulders (8), said shoulders being spacing means for forming said slits and one surface of which forming said surface (11) of the head.

3. A dental mirror according to claim 2, **characterized** in that the brace (6) is provided with a central hole (13) in said surface (11), said boring (26) opening to said hole.

4. A dental mirror according to anyone of the preceding claims, **characterized** in that the boring is divided in three portions, an upper

Fig. 1

Fig. 2

Fig. 3

Fig. 5

Fig. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR-A-2 327 750 (H. HOLSTAD)<br>* Page 2, line 29 - page 3, line 10; page 3, lines 19-37; figures 1,2 *<br>--- | 1 | A 61 B 1/24<br>A 61 C 17/04 |
| A,D | DE-A-2 844 774 (G.A. PEEL)<br>* Page 4, line 20 - page 5, line 14; figures 1,2 *<br>--- | 1,4 | |
| A | GB-A-1 255 719 (B.F. VELLENDER)<br>* Whole document *<br>--- | 1 | |
| A | GB-A- 942 100 (SIC PRODUCTS LTD)<br>* Page 2, lines 74-92; figures 3,4 *<br>----- | 1,2,4 | |

TECHNICAL FIELDS SEARCHED (Int. Cl.4)

A 61 B
A 61 C

The present search report has been drawn up for all claims

| Place of search | Date of completion of the search | Examiner |
|---|---|---|
| THE HAGUE | 17-01-1989 | FERRIGNO, A. |

EPO FORM 1503 03.82 (P0401)